# EUROPEAN PATENT SPECIFICATION

(11) **EP 2 668 298 B1**
(45) Date of publication and mention of the grant of the patent: **11.01.2017**
(21) Application number: 12739930.1
(22) Date of filing: 25.01.2012
(51) Int. Cl.: C12Q 1/68, G01N 33/68, A61K 38/17

(54) **METHODS OF DIAGNOSING AND TREATING AGE-RELATED MACULAR DEGENERATION**
VERFAHREN ZUR DIAGNOSE UND BEHANDLUNG ALTERSBEDINGTER MAKULADEGENERATION
MÉTHODES DE DIAGNOSTIC ET DE TRAITEMENT DE LA DÉGÉNÉRESCENCE MACULAIRE LIÉE À L'ÂGE

(30) Priority: 25.01.2011 US 201161435989 P
(43) Date of publication of application: 04.12.2013
(73) Proprietor: The United States of America, as represented by The Secretary, Department of Health and Human Services, Bethesda, Maryland 20892-7660 (US)
(72) Inventor: WEI, Lai, Rockville, MD 20852 (US); NUSSENBLATT, Robert, Bethesda, MD 20814 (US); LIU, Baoying, North Potomac, MD 20878 (US); CHAN, Chi-chao, Kensington, MD 20895 (US)
(74) Representative: Hallybone, Huw George
(86) International application number: PCT/US2012/022511
(87) International publication number: WO 2012/103187

(56) References cited:
- WO-A1-2007/071439
- WO-A2-2009/105757
- WO-A2-2010/111595
- US-A1- 2004 142 334
- US-A1- 2004 142 334
- US-A1- 2008 118 928
- BROWN ET AL: "Anti-VEGF Agents in the Treatment of Neovascular Age-related Macular Degeneration: Applying Clinical Trial Results to the Treatment of Everyday Patients", AMERICAN JOURNAL OF OPHTHALMOLOGY, ELSEVIER, AMSTERDAM, NL, vol. 144, no. 4, 21 September 2007 (2007-09-21), pages 627-637.e2, XP022264151, ISSN: 0002-9394, DOI: 10.1016/J.AJO.2007.06.039
- WRIGHT JILL F ET AL: "The human IL-17F/IL-17A heterodimeric cytokine signals through the IL-17RA/IL-17RC receptor complex", THE JOURNAL OF IMMUNOLOGY, THE AMERICAN ASSOCIATION OF IMMUNOLOGISTS, US, vol. 181, no. 4, 1 August 2008 (2008-08-01), pages 2799-2805, XP002629394, ISSN: 0022-1767
- SARAH L. GAFFEN: "Structure and signalling in the IL-17 receptor family", NATURE REVIEWS IMMUNOLOGY, vol. 9, no. 8, 1 August 2009 (2009-08-01), pages 556-567, XP055052444, ISSN: 1474-1733, DOI: 10.1038/nri2586
- NUSSENBLATT ET AL: "Age-related Macular Degeneration and the Immune Response: Implications for Therapy", AMERICAN JOURNAL OF OPHTHALMOLOGY, ELSEVIER, AMSTERDAM, NL, vol. 144, no. 4, 21 September 2007 (2007-09-21), pages 618-626.e2, XP022264150, ISSN: 0002-9394, DOI: 10.1016/J.AJO.2007.06.025
- BAOYING LIU ET AL: "Complement component C5a Promotes Expression of IL-22 and IL-17 from Human T cells and its Implication in Age-related Macular Degeneration", JOURNAL OF TRANSLATIONAL MEDICINE, BIOMED CENTRAL, LONDON, GB, vol. 9, no. 1, 15 July 2011 (2011-07-15), page 111, XP021105638, ISSN: 1479-5876, DOI: 10.1186/1479-5876-9-111
- VERITY F. OLIVER ET AL: "Hypomethylation of the IL17RC Promoter in Peripheral Blood Leukocytes Is Not A Hallmark of Age-Related Macular Degeneration", CELL REPORTS, vol. 5, no. 6, 1 December 2013 (2013-12-01), pages 1527-1535, XP055147683, ISSN: 2211-1247, DOI: 10.1016/j.celrep.2013.11.042
- LAI WEI ET AL: "Hypomethylation of the IL17RC Promoter Associates with Age-Related Macular Degeneration", CELL REPORTS, vol. 2, no. 5, 1 November 2012 (2012-11-01), pages 1151-1158, XP055135735, ISSN: 2211-1247, DOI: 10.1016/j.celrep.2012.10.013
- PAUL N BAIRD ET AL: "Age-related macular degeneration and DNA methylation", EPIGENOMICS, vol. 5, no. 3, 1 June 2013 (2013-06-01), pages 239-241, XP055147689, ISSN: 1750-1911, DOI: 10.2217/epi.13.19
- Daniel Ardeljan ET AL: "Interleukin-17 retinotoxicity is prevented by gene transfer of a soluble interleukin-17 receptor acting as a cytokine blocker: implications for age-related macular degeneration", PloS one, 29 April 2014 (2014-04-29), page e95900, XP055135641, United States DOI: 10.1371/journal.pone.0095900 Retrieved from the Internet: URL:http://www.ncbi.nlm.nih.gov/pubmed/247 80906 [retrieved on 2014-08-20]
- WEI L. ET AL.: 'Gene expression of the whole mouse eye' NCBI GENE EXPRESSION OMNIBUS, SERIES GSE28002, [Online] 20 March 2011, XP003032759
- WEI L. ET AL.: 'Epigenetic regulation of IL17RC in age-related macular degeneration (MeDIP-chip)' NCBI GENE EXPRESSION OMNIBUS, SERIES GSE28032, [Online] 21 March 2011, XP003032760

## Description

### TECHNICAL FIELD

The present invention relates to methods of diagnosing the presence, of age-related macular degeneration in a patient. Methods of treating age-related macular degeneration with soluble IL-17RC molecules are also described.

### BACKGROUND

Age-related macular degeneration (AMD) is the most common cause of irreversible blindness in the elder population worldwide. It currently affects more than 1.75 million individuals in the United States alone and this number will increase to almost 3 million in 10 years. AMD leads to progressive loss of central vision because of damage to the retina.

In light of the irreversible nature of the disease, methods of diagnosing AMD, as well as methods of identifying whether a patient is at risk of developing AMD are needed so medical intervention can be initiated as early into the disease as possible.

Currently, anti-Vascular Endothelial Growth Factor (anti-VEGF) drugs are the only available drugs for the treatment of neovascular wet AMD. Campa C, Harding SP. Anti-VEGF Compounds in the Treatment of Neovascular Age Related Macular Degeneration. Curr Drug Targets 2010;2010:1. As such, alternative treatments are needed.

US 2004/142334 describes a method of diagnosing the presence, severity, or predisposition for development of age-related macular degeneration in a patient.

Brown et al. (Am J Opthalmol. 2007 Oct;144(4):627-37) describes a method of treating age-related macular degeneration in a patient comprising administering to the patient a therapeutically effective amount of anti-VEGF agent.

WO 2007/071439 describes soluble IL-17RC molecules for the treatment of inflammatory or autoimmune disorders.

### SUMMARY

The invention provides a method of diagnosing age-related macular degeneration in a patient comprising:
determining the methylation of the promoter of *IL17RC* gene in the DNA from peripheral blood mononuclear cells (PBMCs) isolated from a patient having, suspected of having, or suspected to be at risk for age-related macular degeneration;
determining whether the promoter of *IL17RC* gene is less methylated relative to the methylation of the promoter of *IL17RC* gene in control PBMCs isolated from an individual or individuals not having age-related macular degeneration, the less methylation being indicative of the presence, of age-related macular degeneration in the patient.

The invention provides a method of diagnosing age-related macular degeneration in a patient comprising:
determining the frequency of IL-17RC⁺ cells in a whole blood sample from a patient having, suspected of having, or suspected to be at risk for age-related macular degeneration;
determining whether the blood sample has an elevation in the frequency of IL-17RC⁺ cells relative to the frequency of IL-17RC⁺ cells in a control from an individual or individuals not having age-related macular degeneration, the elevation being indicative of the presence, of age-related macular degeneration in the patient.

The invention provides a soluble IL-17RC molecule for treating age-related macular degeneration.

### BRIEF DESCRIPTION OF THE DRAWINGS

FIG. 1A depicts MeDIP-chip data showing hypo-methylated CpG sites were found in AMD twins but not in non-AMD twins around the promoters of *CCL22* and *IL17RC* genes.
FIG. 1B depicts methylation status on the promoters of *CCL22* and *IL17RC* genes in siblings with discordant AMD.
FIG. 2 depicts that a hypo-methylated promoter of the *IL17RC* gene was found only in AMD patients, as compared to a hyper-methylated promoter of the *IL17RC* gene in healthy controls.
FIG. 3A depicts that increased levels of IL-17RC protein expression are found on several types of cells from AMD patients.
FIG. 3B depicts that increased levels of IL-17RC protein expression are found on CD14⁺ monocyte in an AMD patient.
FIG. 3C depicts that increased levels of IL-17RC expression are found on CD14⁺ monocyte in 28 AMD patients compared to 18 non-AMD controls.
FIG. 4A depicts that elevated expression of CXCR1, CXCR2, and CXCR4 mRNA in sorted IL17RC⁺CD14⁺ monocytes as compared to IL17RC-CD14⁺ monocytes.
FIG. 4B depicts that IL-17RC mRNA is detectable in the retinal and choroidal tissues from AMD patients but not of that from non-AMD controls.

### DETAILED DESCRIPTION OF ILLUSTRATIVE EMBODIMENTS

The present invention relates to methods of diagnosing the presence, of age-related macular degeneration in a patient. According to the invention, these methods include determining the methylation of the promoter of the *IL17RC* gene in the DNA from peripheral blood mononuclear cells (PBMCs) isolated from a patient having, suspected of having, or suspected to be at risk for age-related macular degeneration; and determining whether the promoter of the *IL17RC* gene is less methylated relative to the methylation of the promoter of the *IL17RC* gene in control PBMCs isolated from an individual or individuals not having age-related macular degeneration, the less methylation being indicative of the presence, of age-related macular degeneration in the patient.

In another embodiment, the diagnosis of the presence, of age-related macular degeneration in a patient is accomplished using methods that include determining the frequency of IL-17RC⁺ cells in a whole blood sample from a patient having, suspected of having, or suspected to be at risk for age-related macular degeneration; and determining whether the blood sample has an elevation in the frequency of IL-17RC⁺ cells relative to the frequency of IL-17RC⁺ cells in a control from an individual or individuals not having age-related macular degeneration, the elevation being indicative of the presence, of age-related macular degeneration in the patient.

Nomenclature used herein is consistent with that reported in Wain, H.M. et al. Guidelines for Human Gene Nomenclature Genomics 79(4):464-470 (2002.

As used herein, *"IL17RC"* refers to the IL17RC gene (Gene ID 84818), either DNA or RNA, or the locus of the gene.

As used herein, "IL-17RC" refers to the protein translated from the *IL17RC* gene.

### Difference in DNA Methylation Patterns Between Twins with Discordant AMD

Three pairs of twins with phenotypic discordance of AMD were identified. Their age and gender information is listed in Table 1.

**Table 1: Age and Gender of 212 Patients with AMD and 106 Controls without AMD**

| | **Gender (M/F)** | **Ave. Age** | **CFH rs1061170 (TT/TC/CC/unknown)*** | **ARMS2 rs10490924 (GG/GT/TT/unknown)** | **HTRA1 rs11200638 (GG/GA/AA/unknown)** |
|---|---|---|---|---|---|
| **Twins** | | | | | |
| Non-AMD1** | F | 88 | TC | TT | AA |
| AMD1 | F | 88 | TC | TT | AA |
| Non-AMD2 | F | 78 | CC | GG | GG |
| AMD2 | M | 78 | CC | GT | GA |
| Non-AMD3 | F | 79 | CC | GT | GA |
| AMD3 | F | 79 | CC | GT | GA |
| | | | | | |
| **Siblings** | | | | | |
| Non-AMD | 4/3 | 65.9 | 0/6/1/0 | 2/2/2/1 | 1/2/0/4 |
| AMD | 2/5 | 63.4 | 0/4/3/0 | 0/3/3/1 | 0/1/0/6 |
| | | | | | |
| **Case-Control Patient Cohort** | | | | | |
| Non-AMD | 45/51 | 70.2 | 41/39/16/0 | 61/30/5/0 | 59/29/8/0 |
| AMD | 91/111 | 80.5 | 40/93/67/2 | 57/90/44/11 | 58/89/49/8 |

To investigate the DNA methylation difference between the twins with discordant AMD, genomic DNA was obtained from their peripheral blood mononuclear cells (PBMCs) and subjected to MeDIP-chip analysis using NimbleGen Human DNA Methylation 2.1M Deluxe Promoter Array (Roche-NimbleGen, Madison WI), which includes probes tiling through 7250 bp upstream and 3250 bp downstream of transcription start sites of all genes and microRNAs. A total of 231 genes showed consistent differential methylation patterns on their promoters between AMD twins and their non-AMD co-twins. Consistent with the speculation that AMD might be an immunological disease, our gene ontology enrichment analysis using Ingenuity Systems (Redwood City, CA; http://www.ingenuity.com/) identified "Immunological Disease" as one of the 5 most significantly enriched gene categories among the list of genes with differential DNA methylation patterns between twins (Table 2).

**Table 2: Enrichment of Disease Categories Among Genes with Differential Promoter Methylation Patterns Between AMD and non-AMD Twins**

| **Diseases and Disorders Name** | **Corrected P value** | **# of Molecules in list** |
|---|---|---|
| immunological Disease | 4.13E-02 | 58 |
| Gastrointestinal | 4.13E-02 | 78 |
| Endocrine System Disorders | 4.13E-02 | 55 |
| Metabolic Disease | 4.13E-02 | 63 |
| Genetic Disorder | 4.19E-02 | 127 |

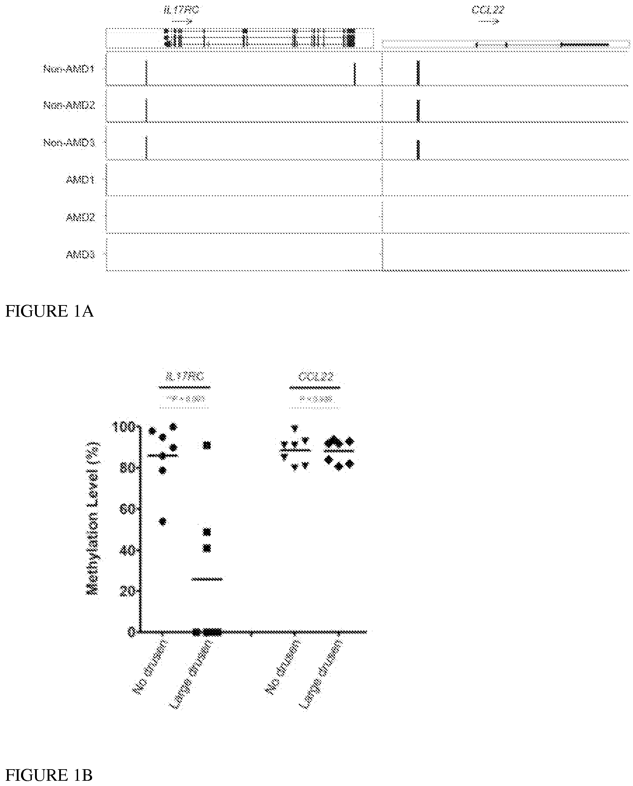

Elevated serum levels of Th17 cytokines, IL-17A and IL-22, in AMD patients (32) were demonstrated. In addition, a previous study has indicated the induction of IL-17 by complement C5a (33). Therefore, among our list of 231 genes with differential methylation patterns between twin pairs, focus was on molecules contributing to Th17 immunity. Intriguingly, as shown in Figure 1A, MeDIP-chip data suggested that methylated CpG sites were only found in the twins without AMD but not in their AMD co-twins in the promoter regions of IL17RC, which encodes the receptor for IL-17A and IL-17F dimers, as well as CCL22, which is the chemokine mediating trafficking of activated T lymphocytes to inflammatory sites.

To test whether a similar DNA methylation pattern could be found in other AMD patients with more diverse genetic backgrounds, 7 pairs of siblings were identified who had discordant AMD phenotypes (for all sibling pairs, one of the two had large drusen greater than 125 micron in diameter in at least one eye, and the other had no drusen). Methyl-Profiler assays were carried out to detect the methylation status on the promoters of IL17RC and CCL22 (Figure 1B). Intriguingly, a significant differential methylation status between siblings with discordant AMD was found on the promoter of IL17RC (P=0.003, Mann-Witney test). A hypomethylated IL17RC promoter was only found in the DNA from the siblings with AMD similar to that observed in the discordant twins.

### Hypo-methylated Promoter if the IL17RC gene in AMD Patients

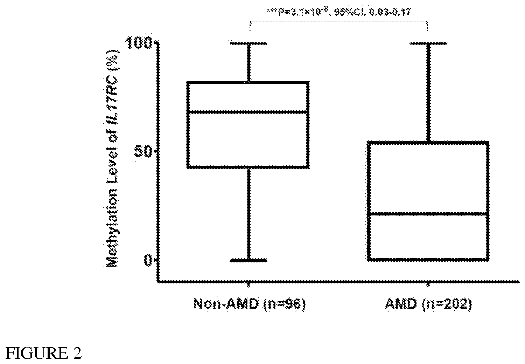

To investigate the methylation status of IL17RC promoters in patients and healthy controls who do not share known genetic similarities, genomic DNA was collected from the PBMCs of 202 AMD patients (95 wet-AMD and 107 dry-AMD) as well as 96 healthy individuals without AMD (non-AMD). The average age of AMD patients and non-AMD controls were 80.5 and 70.2 years old, respectively (Table 1, Case-Control Patient Cohort). Similar to the findings with both twins and siblings, a hypomethylated IL17RC promoter was also found in AMD patients, as compared to a hypermethylated IL17RC promoter in non-AMD controls (95% CI, 0.03-0.17, P=3.1x10⁻⁸, multivariable logistic regression adjusted for age and gender) (Figure 2). Collectively, general association was shown of a hypomethylated IL17RC promoter with AMD.

### Elevated expression of IL-17RC in AMD patients' peripheral blood

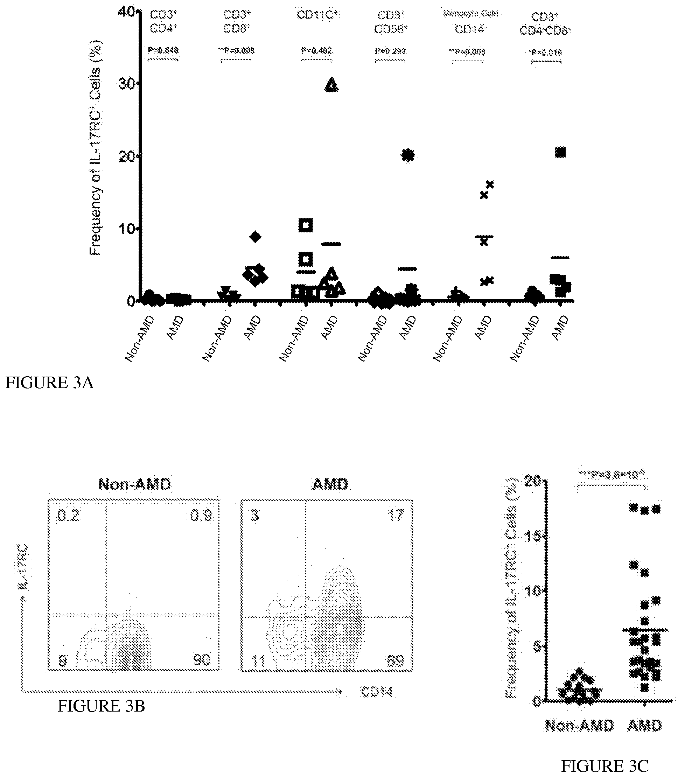

DNA methylation generally associates with gene silencing. To test whether DNA hypomethylation found on the promoter of IL17RC resulted in gene de-silencing and hence increased expression in only AMD patients, FACS analysis of whole blood samples was performed, together with other cell surface markers identifying peripheral hematopoietic cell lineages. Increased levels of IL-17RC expression were found on several types of cells from AMD patients as compared to age matched non-AMD controls, including CD3⁺CD4⁻CD8⁻ and CD8⁺ T cells, but not on CD4⁺ T cells (Figure 3A). Intriguingly, a significantly elevated frequency of IL-17RC⁺CD14⁺ monocytes in the peripheral blood of both dry and wet AMD patients was found (Figure 3B and 3C). These data suggest that the hypomethylation of IL17RC promoter results in the elevated expression of the IL-17RC protein on selected cells in peripheral blood of AMD patients.

### Elevated expression of IL17RC in AMD patients' eyes

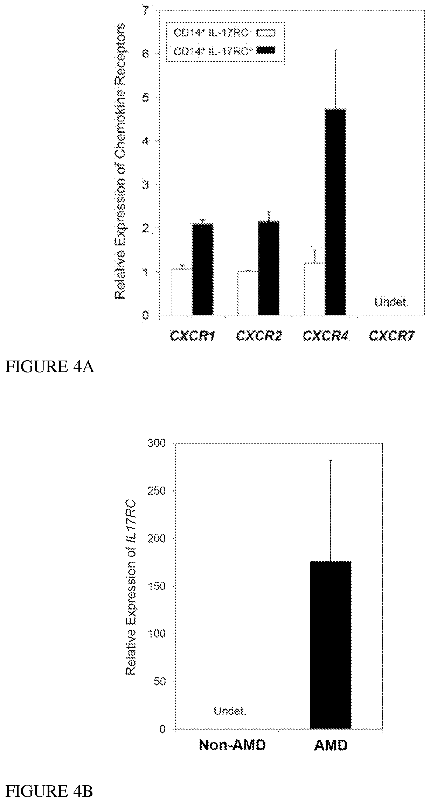

To investigate the characteristics of IL-17RC⁺CD14⁺ monocytes found only in AMD patients, the expression of CXCR4 and CXCR7, which encode the receptors for CXCL12, as well as the expression of CXCR1 and CXCR2, which encode the receptors for IL-8, in the sorted IL-17RC⁺CD14+ monocytes comparing to the sorted IL-17RC⁻CD14⁺ monocytes. As shown in Fig. 4A, the expression of CXCR1 and CXCR2 in IL-17RC⁺CD14⁺ monocytes were increased by 2 fold as compared to IL-17RC⁺CD14⁻ monocytes, while the expression of CXCR4 in IL-17RC+CD14+ monocytes was over 4 fold higher than its expression in IL-17RC⁻CD14⁺ monocytes. The expression of CXCR7 was undetectable in either of these cells. These data suggest that IL-17RC⁺CD14⁺ monocytes may have a stronger potential to migrate into the eye than the IL-17RC⁺CD14⁻ monocytes.

To test whether IL17RC expression could be found in the retinal and choroidal tissues of AMD patients, formalin-fixed, paraffin-embedded (FFPE) archived retina slides were obtained from two patients with the wet form of AMD as well as two age-matched control eyes. Quantitative real-time PCR was performed to detect the expression of IL17RC mRNA. Interestingly, IL17RC was only detectable in the retina and choroid of AMD patients but not the non-AMD controls (Fig. 4B). Thus elevated IL17RC expression was found not only in the peripheral blood but also in the eyes of AMD patients.

Twin and sibling designs are broadly used in studies dissecting the genetic versus environmental contributions to various diseases. Early studies using small patient cohorts indicated a significantly higher concordance rate of AMD in monozygotic than in dizygotic twins or families and strongly suggest a genetic predisposition to AMD. *See* Meyers SM. A twin study on age-related macular degeneration. Trans Am Ophthalmol Soc 1994;92:775-843; Gottfredsdottir MS, Sverrisson T, Musch DC, Stefansson E. Age related macular degeneration in monozygotic twins and their spouses in Iceland. Acta Ophthalmol Scand 1999;77:422-5; Klein ML, Mauldin WM, Stoumbos VD. Heredity and age-related macular degeneration. Observations in monozygotic twins. Arch Ophthalmol 1994;112:932-7; Heiba IM, Elston RC, Klein BE, Klein R. Sibling correlations and segregation analysis of age-related maculopathy: the Beaver Dam Eye Study. Genet Epidemiol 1994;11:51-67. In two recent twin studies, Hammond et al. showed that the concordance for AMD in monozygotic twins was 0.37 compared with 0.19 in dizygotic twins, while Seddon et al. suggested that genetic factors can explain 46% to 71% of the variation in the overall severity of AMD. Hammond CJ, Webster AR, Snieder H, Bird AC, Gilbert CE, Spector TD. Genetic influence on early age-related maculopathy: a twin study. Ophthalmology 2002;109:730-6; Seddon JM, Cote J, Page WF, Aggen SH, Neale MC. The US twin study of age-related macular degeneration: relative roles of genetic and environmental influences. Arch Ophthalmol 2005;123:321-7. However, the later study also elucidated that for specific macular drusen and retinal pigment epithelial characteristics, both significant genetic (0.26-0.71) and unique environmental (0.28-0.64) proportions of variance were detected. The three pairs of twins (one monozygotic and two dizygotic) in the present studies display a significant difference in clinical manifestation, providing a unique chance to study the epigenetic difference between them. The present MeDIP-chip analysis identified difference in ∼1.5% of the total CpG sites within 231 gene promoters between AMD twins (data not shown). The results also suggest that epigenetic modifications of DNA may be crucial for understanding the molecular basis of complex diseases like AMD.

AMD has been traditionally considered as a neurodegenerative disease. Recent discovery of association between AMD and SNPs in genes involving in immune responses such as *CFH, CFB, CFI, C3, ARMS2*/*HTRA1, TIMP3, TLR3,* and *TLR4* argues that disregulated immune system may play an important role during onset and sustain of AMD pathology. Swaroop A, Chew EY, Rickman CB, Abecasis GR. Unraveling a multifactorial late-onset disease: from genetic susceptibility to disease mechanisms for age-related macular degeneration. Annu Rev Genomics Hum Genet 2009;10:19-43. Interestingly, the list of genes with differential methylation patterns on their promoters between discordant AMD twins (data not shown), identified by the present MeDIP-chip analysis, also suggested potential involvement of immune system in AMD etiology.

IL-17RC protein is found highly expressed on endothelium and epithelium of lung, intestine, kidney, prostate, and joint. However, it is not clear what types of cells express IL-17RC in human peripheral blood or eyes. IL-17RC protein serves as an essential subunit of IL-17 receptor complex that mediates the signal transduction and inflammatory activities of IL-17A and IL-17F. Its binding affinity and ligand preference, as well as tissue specific distribution are slightly different between the murine and human systems. Recent studies have suggested that high levels of IL-17RC protein expression are found in prostate cancer cells, as well as synoviocytes and blood cells from patients with rheumatoid arthritis. In the present studies, hypomethylated IL17RC promoter was found in patients with both wet and dry AMD as compared to non-AMD controls, which resulted in the increased expression of its protein in CD14⁺ monocytes. Moreover, it was demonstrated that IL-17RC⁺CD14⁺ monocytes expressed elevated CXCR4, CXCR1 and CXCR2, which may promote their trafficking into the inflammatory sites within retinal and choroidal tissues in AMD patients and eventually causing the pathology of AMD. Therefore, these results suggest a potential mechanism by which proinflammatory monocytes could promote AMD pathology.

It has heretofore been identified that DNA hypo-methylation of promoter of the *IL17RC* gene in AMD patients leads to elevated expression of its protein and mRNA in peripheral blood and retina tissues. Thus, promoter DNA methylation pattern and expression of IL-17RC protein can serve as a biomarker for AMD diagnosis. Currently, anti-Vascular Endothelial Growth Factor (anti-VEGF) drugs is the only available drug for the treatment of neovascular wet AMD. The present studies suggest that soluble IL-17RC molecules, which are known in the art, for example, such as those soluble IL-17RC molecules described in U.S. Published Application No. 2008/0305078, which could act as decoy receptors to dampen signalling of IL-17 cytokines, can be a new therapy for AMD. Thus, one aspect of the disclosure involves methods of treating age-related macular degeneration in a patient comprising administering to the patient a therapeutically effective amount of a soluble IL-17RC molecule.

As used herein, a "therapeutically effective amount" is an amount effective, at dosages, and for periods of time necessary, to achieve the desired result with respect to the treatment of the relevant disorder. It will be appreciated that the effective amount of soluble IL-17RC will vary from patient to patient not only with the particular compound, component or composition selected, the route of administration, and the ability of the components to elicit a desired response in the individual, but also with factors such as the disease state or severity of the condition to be alleviated, hormone levels, age, sex, weight of the individual, the state of being of the patient, and the severity of the pathological condition being treated, concurrent medication or special diets then being followed by the particular patient, and other factors which those skilled in the art will recognize, with the appropriate dosage ultimately being at the discretion of the attendant physician. Dosage regimens may be adjusted to provide the improved therapeutic response. An effective amount is also one in which any toxic or detrimental effects of the components are outweighed by the therapeutically beneficial effects. As an example, the soluble IL-17RC molecules of the present invention may be administered at a dosage and for a time such that the level of activation and adhesion activity of platelets is reduced as compared to the level of activity before the start of treatment.

The soluble IL-17RC molecules of the invention may be administered in an effective amount by any of the conventional techniques well-established in the medical field. The soluble IL-17RC molecules of the invention may be administered by any means that results in the contact of the active agents with the agents' site or sites of action in the body of a patient. The soluble IL-17RC molecules may be administered by any conventional means available.

### EXPERIMENTAL SECTION

### Methods

### Patients

All protocols were approved by institutional review boards, and written informed consent for epigenetic and genetic testing and participation was provided by the patients to the National Institutes of Health (NIH), Casey Eye Institute (CEI) (Oregon Health and Science University), or Centre for Eye Research Australia (CERA) through the Royal Victorian Eye and Ear Hospital (RVEEH) Human Research and Ethics Committee and the Australian Twin Registry (ATR) approved the project. Twin volunteers, both identical and non-identical with or without known eye disease, of either gender, aged 18 years or older were invited to participate through the ATR. The non-AMD controls were from the NIH eye clinics and were screened for AMD. All patients and non-AMD controls are Caucasian. The age and gender information was listed in Table 1.

### DNA Methylation Microarray (MEDIP-chip) and Data Analysis

DNA sample preparation and hybridization to DNA methylation microarray were performed according to the manufacture's protocols provided by Roche-NimbleGen, WI. Briefly, Genomic DNA is isolated from peripheral blood mononuclear cells (PBMCs) using the Qiagen DNeasy kit (Qiagen, CA). Six µg total DNA was then digested with *MseI* endonuclease (NEB, MA) overnight to produce small fragments (200-1000bp). Antibody against 5-methyl cytidine (Ab10805, Abcam, MA) was used to immunoprecipitate methylated DNA fragments. Enriched methylated DNA and input DNA was amplified using WGA2 GenomePlex Complete Whole Genome Amplification Kit (Sigma, MO) according to manufacture's protocol and sent to Roche-NimbleGen for labelling and hybridization to Human DNA Methylation 2.1M Deluxe Promoter Arrays. Data analysis service was provided by NimbleGen. All raw data were deposited in GEO with accession number GSE28033.

### Detection of DNA Methylation on Selected Gene Promoters

One µg total genomic DNA from PBMCs was digested for 6 hours at 37 °C using the Methyl-Profiler DNA Methylation Enzyme Kit (SABiosciences, MD) according to the manufacture's instruction. Quantitative Real-time PCR was performed with an ABI PRISM 7900HT Sequence Detection System (Applied Biosystems, CA). The percentage of methylated DNA was calculated by the difference of CT cycle numbers between samples with total undigested DNA and methylation-sensitive restriction enzyme digested DNA. The following primers were used:
*CCL22F:* ACCACCACACCCAGCTAAAT,
*CCL22R:* CACGCCCTGCT-CATTTTTAT;
*IL17RCF:* CCAAGATCCGGAAAGATGAA,
*IL17RCR:* GCATCT-TCTAGGTGCCAGGT.

### Detection of Cell Surface Molecules by Flow Cytometry

Whole blood, collected in Na-heparin vacutainers (BD, CA), from AMD patients or healthy volunteers were first subjected to Red Blood Cell lysis using ACK Lysis buffer (Quality Biological Inc, MD). Cells were then incubated in sterile PBS supplemented with 10% FCS and fluorochrome-conjugated antibodies against CD3, CD4, CD8, CD14, CD45, CD11C, CD56 (all from BD, CA) as well as IL-17RC (FAB22691A, R&D, MN), for 30 minutes at room temperature in dark. Cells were washed three times and analyzed on LSR-II analyzer (BD, CA). All flow cytometry data were analysed using FlowJo 9.1 (Treestar, OR).

### Detection of IL17RC Expression in Human Retinal and Choroidal Tissues

Archived formalin-fixed paraffin-embedded (FFPE) samples were first washed in 100% Xylene for 10 times, followed by sequential washes in 100% ethanol, 95% ethanol, 75% ethanol, and water (10 times in each solution). Total RNA was isolated from retina tissues of two AMD patients as well as two age-matched controls, using mirVana miRNA kit (Ambion, TX). Reverse transcription of RNA was performed for 12 hours at 37 °C, followed by Quantitative Real-time PCR, using the primers and probes for *18s rRNA* (Hs03928990_g1) and *IL17RC* (Hs00994305_ml) from Applied Biosystems. The comparative threshold cycle method and *18s rRNA* was used to normalize the expression of the *IL17RC.*

### Genetic Analysis

Genomic DNA was extracted from PBMCs of each individual using the Genomic DNA Purification kit (Promega, WI). DNA samples were analyzed by TaqMan genotyping assays using the ABI PRISM 7500 sequence detection system (Applied Biosystems, CA). The primers and probes for *ARMS2* rs 10490924 was from the inventory SNP assay while *CFH* rs 1061170 and *HTRA1* rs11200638 were custom-designed from Applied Biosystems. Genotypes were determined based on the fluorescence intensities of FAM and VIC. The call rates of 3 assays were >98.5% and the call accuracies (consistency of duplicate wells of selected samples) were 100%.

### Statistical Analysis

Statistical analysis was performed using GraphPad Prism 5.0 software (GraphPad, La Jolla, CA). The Mann-Whitney test was used to compare differences between two groups and the significance level was set at P<0.05. Multivariable logistic regression analysis was used to detect the difference of the IL17RC promoter methylation status, adjusted by age, gender, genotypes of CFH, ARMS2, or HTRA1, using SAS 9.2. The significance level was set at P<0.05.

### References

1. Friedman DS, O'Colmain BJ, Munoz B, et al. Prevalence of age-related macular degeneration in the United States. Arch Ophthalmol 2004;122:564-72.
2. Swaroop A, Chew EY, Rickman CB, Abecasis GR. Unraveling a multifactorial late-onset disease: from genetic susceptibility to disease mechanisms for age-related macular degeneration. Annu Rev Genomics Hum Genet 2009;10:19-43.
3. Edwards AO, Ritter R, 3rd, Abel KJ, Manning A, Panhuysen C, Farrer LA. Complement factor H polymorphism and age-related macular degeneration. Science
4. Haines JL, Hauser MA, Schmidt S, et al. Complement factor H variant increases the risk of age-related macular degeneration. Science 2005;308:419-21.
5. Klein RJ, Zeiss C, Chew EY, et al. Complement factor H polymorphism in age-related macular degeneration. Science 2005;308:385-9.
6. Hageman GS, Anderson DH, Johnson LV, et al. A common haplotype in the complement regulatory gene factor H (HF1/CFH) predisposes individuals to age-related macular degeneration. Proc Natl Acad Sci U S A 2005;102:7227-32.
7. Maller J, George S, Purcell S, et al. Common variation in three genes, including a noncoding variant in CFH, strongly influences risk of age-related macular degeneration. Nat Genet 2006;38:1055-9.
8. Gold B, Merriam JE, Zernant J, et al. Variation in factor B (BF) and complement component 2 (C2) genes is associated with age-related macular degeneration. Nat Genet 2006;38:458-62.
9. Yates JR, Sepp T, Matharu BK, et al. Complement C3 variant and the risk of age-related macular degeneration. N Engl J Med 2007;357:553-61.
10. Fagerness JA, Maller JB, Neale BM, Reynolds RC, Daly MJ, Seddon JM. Variation near complement factor I is associated with risk of advanced AMD. Eur J Hum Genet 2009;17:100-4.
11. Dewan A, Liu M, Hartman S, et al. HTRA1 promoter polymorphism in wet age-related macular degeneration. Science 2006;314:989-92.
12. Yang Z, Camp NJ, Sun H, et al. A variant of the HTRA1 gene increases susceptibility to age-related macular degeneration. Science 2006;314:992-3.
13. Fritsche LG, Loenhardt T, Janssen A, et al. Age-related macular degeneration is associated with an unstable ARMS2 (LOC387715) mRNA. Nat Genet 2008;40:892-6.
14. Kanda A, Chen W, Othman M, et al. A variant of mitochondrial protein LOC387715/ARMS2, not HTRA1, is strongly associated with age-related macular degeneration. Proc Natl Acad Sci U S A 2007;104:16227-32.
15. Baird PN, Richardson AJ, Robman LD, et al. Apolipoprotein (APOE) gene is associated with progression of age-related macular degeneration (AMD). Hum Mutat 2006;27:337-42.
16. Chen W, Stambolian D, Edwards AO, et al. Genetic variants near TIMP3 and high-density lipoprotein-associated loci influence susceptibility to age-related macular degeneration. Proc Natl Acad Sci U S A 2010;107:7401-6.
17. Zareparsi S, Buraczynska M, Branham KE, et al. Toll-like receptor 4 variant D299G is associated with susceptibility to age-related macular degeneration. Hum Mol Genet 2005;14:1449-55.
18. Yang Z, Stratton C, Francis PJ, et al. Toll-like receptor 3 and geographic atrophy in age-related macular degeneration. N Engl J Med 2008;359:1456-63.
19. Neale BM, Fagerness J, Reynolds R, et al. Genome-wide association study of advanced age-related macular degeneration identifies a role of the hepatic lipase gene (LIPC). Proc Natl Acad Sci U S A 2010;107:7395-400.
20. Peter I, Seddon JM. Genetic epidemiology: successes and challenges of genome-wide association studies using the example of age-related macular degeneration. Am J Ophthalmol 2010;150:450-2 e2.
21. de Jong PT. Age-related macular degeneration. N Engl J Med 2006;355:1474-85.
22. Javierre BM, Fernandez AF, Richter J, et al. Changes in the pattern of DNA methylation associate with twin discordance in systemic lupus erythematosus. Genome Res 2009;20:170-9.
23. Baranzini SE, Mudge J, van Velkinburgh JC, et al. Genome, epigenome and RNA sequences of monozygotic twins discordant for multiple sclerosis. Nature 2010;464:1351-6.
24. Kaminsky ZA, Tang T, Wang SC, et al. DNA methylation profiles in monozygotic and dizygotic twins. Nat Genet 2009;41:240-5.
25. Fraga MF, Ballestar E, Paz MF, et al. Epigenetic differences arise during the lifetime of monozygotic twins. Proc Natl Acad Sci U S A 2005;102:10604-9.
26. Meyers SM. A twin study on age-related macular degeneration. Trans Am Ophthalmol Soc 1994;92:775-843.
27. Gottfredsdottir MS, Sverrisson T, Musch DC, Stefansson E. Age related macular degeneration in monozygotic twins and their spouses in Iceland. Acta Ophthalmol Scand 1999;77:422-5.
28. Klein ML, Mauldin WM, Stoumbos VD. Heredity and age-related macular degeneration. Observations in monozygotic twins. Arch Ophthalmol 1994;112:932-7.
29. Heiba IM, Elston RC, Klein BE, Klein R. Sibling correlations and segregation analysis of age-related maculopathy: the Beaver Dam Eye Study. Genet Epidemiol 1994;11:51-67.
30. Hammond CJ, Webster AR, Snieder H, Bird AC, Gilbert CE, Spector TD. Genetic influence on early age-related maculopathy: a twin study. Ophthalmology
31. Seddon JM, Cote J, Page WF, Aggen SH, Neale MC. The US twin study of age-related macular degeneration: relative roles of genetic and environmental influences. Arch Ophthalmol 2005;123:321-7.
32. Chang SH, Dong C. Signaling of interleukin-17 family cytokines in immunity and inflammation. Cell Signal;2010:2.
33. Ho AW, Gaffen SL. IL-17RC: a partner in IL-17 signaling and beyond. Semin Immunopathol 2009;32:33-42.
34. Ge D, You Z. Expression of interleukin-17RC protein in normal human tissues. Int Arch Med 2008;1:19.
35. Campa C, Harding SP. Anti-VEGF Compounds in the Treatment of Neovascular Age Related Macular Degeneration. Curr Drug Targets 2010;2010:1.
36. Group TA-REDSR. A randomized, placebo-controlled, clinical trial of high-dose supplementation with vitamins C and E, beta carotene, and zinc for age-related macular degeneration and vision loss: AREDS report no. 8. Arch Ophthalmol 2001;119:1417-36.
37. Krishnadev N, Meleth AD, Chew EY. Nutritional supplements for age-related macular degeneration. Curr Opin Ophthalmol 2010;21:184-9.

## Claims

1. A method of diagnosing age-related macular degeneration in a patient
comprising:
determining the methylation of the promoter of *IL17RC* gene in the DNA from peripheral blood mononuclear cells (PBMCs) isolated from a patient having, suspected of having, or suspected to be at risk for age-related macular degeneration;
determining whether the promoter of *IL17RC* gene is less methylated relative to the methylation of the promoter of *IL17RC* gene in control PBMCs isolated from an individual or individuals not having age-related macular degeneration, the less methylation being indicative of the presence, of age-related macular degeneration in the patient.

2. A method of diagnosing age-related macular degeneration in a patient
comprising:
determining the frequency of IL-17RC⁺ cells in a whole blood sample from a patient having, suspected of having, or suspected to be at risk for age-related macular degeneration;
determining whether the blood sample has an elevation in the frequency of IL-17RC⁺ cells relative to the frequency of IL-17RC⁺ cells in a control from an individual or individuals not having age-related macular degeneration, the elevation being indicative of the presence, of age-related macular degeneration in the patient.

3. A soluble IL-17RC molecule for use in the treatment of age-related macular degeneration.

## Patentansprüche

1. Verfahren zum Diagnostizieren von altersbedingter Makuladegeneration in einem Patienten, umfassend:
Bestimmen der Methylierung des Promotors des *IL17RC-*Gens in der DNA aus peripheren mononukleären Blutzellen (PBMCs), die aus einem Patienten isoliert wurden, der altersbedingte Makuladegeneration hat, unter dem Verdacht steht, dass er diese Krankheit hat, oder unter dem Verdacht steht, dass er einem Risiko für diese Krankheit unterliegt;
Bestimmen, ob der Promotor des *IL17RC*-Gens im Vergleich zu der Methylierung des Promotors des *IL17RC-*Gens in Kontroll-PBMCs, die aus einem Individuum oder Individuen isoliert wurden, das/die keine altersbedingte Makuladegeneration hat/haben, schwächer methyliert ist, wobei die schwächere Methylierung das Vorliegen einer altersbedingten Makuladegeneration in dem Patienten anzeigt.

2. Verfahren zum Diagnostizieren von altersbedingter Makuladegeneration in einem Patienten, umfassend:
Bestimmen der Häufigkeit von IL-17RC⁺-Zellen in einer Vollblutprobe aus einem Patienten, der altersbedingte Makuladegeneration hat, unter dem Verdacht steht, dass er diese Krankheit hat, oder unter dem Verdacht steht, dass er einem Risiko für diese Krankheit unterliegt;
Bestimmen, ob die Blutprobe eine Erhöhung der Häufigkeit von IL-17RC⁺-Zellen im Vergleich zur Häufigkeit von IL-17RC⁺-Zellen in einer Kontrolle aus einem Individuum oder Individuen, das/die keine altersbedingte Makuladegeneration hat/haben, aufweist, wobei die Erhöhung das Vorliegen einer altersbedingten Makuladegeneration in dem Patienten anzeigt.

3. Lösliches IL-17RC-Molekül zur Verwendung bei der Behandlung von altersbedingter Makuladegeneration.

## Revendications

1. Procédé pour le diagnostic de la dégénérescence maculaire liée à l'âge chez un patient, consistant à :
déterminer la méthylation du promoteur du gène IL17RC dans l'ADN de cellules mononuclées de sang périphérique (PBMC) isolées à partir d'un patient présentant, suspecté de présenter ou suspecté de présenter un risque pour une dégénérescence maculaire liée à l'âge ;
déterminer si le promoteur du gène IL17RC est moins méthylé par rapport à la méthylation du promoteur du gène IL17RC dans des PBMC témoin isolées à partir d'un individu ou d'individus ne présentant pas de dégénérescence maculaire liée à l'âge, la méthylation inférieure étant indicatrice de la présence de la dégénération maculaire liée à l'âge chez le patient.

2. Procédé pour le diagnostic de la dégénérescence maculaire liée à l'âge chez un patient consistant à :
déterminer la fréquence de cellules IL-17RC⁺ dans un échantillon de sang complet provenant d'un patient présentant, suspecté de présenter ou suspecté de présenter un risque pour une dégénérescence maculaire liée à l'âge ;
déterminer si l'échantillon sanguin présente une élévation de la fréquence de cellules IL-17RC⁺ par rapport à la fréquence de cellules IL-17RC⁺ dans un témoin provenant d'un individu ou d'individus ne présentant pas de dégénérescence maculaire liée à l'âge, l'élévation étant indicatrice de la présence de la dégénérescence maculaire liée à l'âge chez le patient.

3. Molécule soluble d'IL-17RC pour une utilisation dans le traitement de la dégénérescence maculaire liée à l'âge.
